# EUROPEAN PATENT SPECIFICATION

(11) **EP 1 604 659 B1**
(45) Date of publication and mention of the grant of the patent: **03.09.2008**
(21) Application number: 04717765.4
(22) Date of filing: 05.03.2004
(51) Int. Cl.: A61K 31/198, A61K 31/401, A61K 9/20, A61K 47/30, A61K 47/38, A61P 3/02, A23L 1/305, A23L 1/29

(54) **TABLET CONTAINING WATER-ABSORBING AMINO ACID**
TABLETTE MIT WASSERABSORBIERENDER AMINOSÄURE
COMPRIME CONTENANT UN ACIDE AMINE ABSORBANT L'EAU

(30) Priority: 06.03.2003 JP 2003059884
(43) Date of publication of application: 14.12.2005
(73) Proprietor: KYOWA HAKKO KOGYO CO., LTD., Tokyo 100-8185 (JP)
(72) Inventor: OHTA, Motohiro c/o Pharmaceutical Research Inst. Kyowa Hakko Kogyo Co., Ltd., Sunto-gun, Shizuoka 4118731 (JP); MORIMOTO, Kiyoshi, Mishima-shi, Shizuoka 4110808 (JP); KUBOYAMA, Muneko c/o Head Office Kyowa Hakko Kogyo Co., Ltd., Chiyoda-ku, Tokyo 1008185 (JP); YOSHIMOTO, Hirokazu c/o Fuji Plant Kyowa Hakko Kogyo Co., Ltd., Sunto-gun, Shizuoka 4118731 (JP); KAWABE, Hideo c/o Head Office Kyowa Hakko Kogyo Co., Ltd., Chiyoda-ku, Tokyo 1008185 (JP)
(74) Representative: Vossius & Partner
(86) International application number: PCT/JP2004/002882
(87) International publication number: WO 2004/078171

(56) References cited:
- EP-A1- 0 447 297
- EP-A2- 0 184 999
- JP-A- 2 049 721
- JP-A- 4 334 317
- JP-A- 6 256 170
- JP-A- 60 196 166
- JP-A- 2003 063 953
- US-A- 4 908 214
- US-A1- 2001 002 269
- DATABASE BIOSIS [Online] BIOSCIENCES INFORMATION SERVICE, PHILADELPHIA, PA, US; 1984, MININA S A ET AL: "COATING GLUTAMIC-ACID TABLETS WITH SHELLAC BASED MATERIAL" XP002374548 Database accession no. PREV198579043231 & KHIMIKO-FARMATSEVTICHESKII ZHURNAL, vol. 18, no. 2, 1984, pages 215-219, ISSN: 0023-1134
- DATABASE BIOSIS [Online] BIOSCIENCES INFORMATION SERVICE, PHILADELPHIA, PA, US; February 2000 (2000-02), YOSHIMARU TETSURO ET AL: "Microencapsulation of L-Lysine for improving the balance of amino acids in ruminants" XP002374549 Database accession no. PREV200000225236 & JOURNAL OF THE FACULTY OF AGRICULTURE KYUSHU UNIVERSITY, vol. 44, no. 3-4, February 2000 (2000-02), pages 359-365, ISSN: 0023-6152
- DATABASE FSTA [Online] INTERNATIONAL FOOD INFORMATION SERVICE (IFIS), FRANkFURT-MAIN, DE; 1972, ANONYMOUS: "Einsatz von Zein in der Lebensmittelindustrie." XP002374550 Database accession no. 72-4-12-m1406 & SÜSSWAREN, vol. 16, no. 11, 1972, page 496,

## Description

The present invention relates to a tablet containing a water-absorbent amino acid, which is used for nutritional supplements or pharmaceuticals, and a method for manufacturing the same.

The muscle fatigue-reducing effect and the fatty acid burning effect of water-absorbent amino acids have been appreciated by consumers, and demands for amino acid-containing nutritious food for sports, dieting, etc. or amino acid-containing pharmaceuticals have been sharply increasing. In order to suit such purposes, it is preferable to provide tablets containing high content of water-absorbent amino acids as nutritious food or the like. However, with respect to water-absorbent amino acids, tablets containing high content of water-absorbent amino acids have not been manufactured hitherto because of their high water-absorbability.

On the other hand, with respect to pharmaceuticals or the like, there are known a method of coating an active ingredient with an enteric polymer in order to reduce the bitter taste [e.g. see JP-A-2000-169364 (corresponding to WO 2000/18372 pamphlet)], a method of coating amino acids with animal fat or the like to absorb amino acids effectively from the fourth stomach in ruminant animals (e.g. see JP-A-1995-289172(corresponding to US5676966)), a method of coating branched chain amino acids with binding solution for granulation to reduce odor and bitter tastes (e.g. Japanese patent No. 3341768 (corresponding to WO 2003/63854 pamphlet)), and the like.

Khimiko-Farmatsevticheskii Zhurnal 18(2) 215-219, 1984 discloses glutamic acid tablets with a shellac based coating. Journal of the Faculty of Agriculture Kyushu University 44 (3-4) 359-365, 2000 teaches about certain amino acid granules coated with zein or shellac.

EP 0477 297 discloses certain amino acid granules coated with zein.

Since amino acids such as proline, lysine, histidine, cysteine, ornithine, glutamic acid, and arginine have an intensive water-absorbability differing from branched chain amino acids, glutamine, etc., the storage stability and tableting property of these amino acids are bad, and thus the blending content in tablets is restricted. Therefore, the improvement of such preparations is desired.

It is an object of the present invention to provide a tablet which can contain high content of water-absorbent amino acid(s).

Thus, the present invention relates to:
1. A compression-molded tablet comprising water-absorbent amino acid granules coated with shellac or zein as a coating agent, wherein the water-absorbent amino acid is at least one amino acid seceded from proline, lysine, histidine, cysteine, ornithine, glutamic acid and arginine.
2. The tablet according to claim 1, wherein the tablet comprises the water-absorbent amino acid in an amount of 10 to 70 % by mass of the total tablet mass.
3. The tablet according to claim 1 or 2, wherein the tablet comprises the coating agent in an amount of 1 to 6 % by mass of the total mass of the granules.
4. The tablet according to any one of claims 1 to 3, wherein the tablet is a chewable preparation.
5. A method for manufacturing a tablet containing a water-absorbent amino acid, which comprises a step of granulating a powder containing a water-absorbent amino acid, wherein the water-absorbent amino acid is at least one amino acid selected from proline, lysine, histidine, cysteine, ornithine, glutamic acid and arginine, coating the resulting granules by spraying an ethanol solution containing 5 to 20 by mass of shellac or zein as a coating agent, and compression molding the resulting coated granules.
6. The method according to claim 5, wherein the water-absorbent amino acid is used in an amount of 10 to 70 % by mass of the total tablet mass.
7. The method according to claim 5 or 6, wherein the coating agent is used in an amount of 1 to 6 % by mass of the total mass of the granules.
8. The tablet according to any of claims 1 to 4 for reducing muscular-fatigue.
9. The tablet according to any of claims 1 to 4 for burning fatty acid.
10. The tablet according to any of claims 1 to 4 for dieting.

The tablet of the present invention comprises water-absorbent amino acid(s) and a coating agent which is soluble in ethanol and hardly soluble in water.

The present invention provides tablets which can contain high content of water-absorbent amino acid(s). The water-absorbent amino acids contained in the tablet of the present invention have effects of muscular-fatigue reduction, fatty acid burn and dieting. Consequently, the tablet can be taken easily as an amino acid preparation for sports, dieting, or pharmaceuticals. In addition, such tablets can be utilized as nutritional supplements, pharmaceuticals and the like in order to supply nutritious of amino acids effective to human health maintenance, or enhance physiological activity of human tissues.

"A water-absorbent amino acid" used in the tablet of the present invention refers to an amino acid having such properties as high absorbability of water used in the manufacture of preparations, high absorbability of moisture and humidity in the air, or deliquescent, adhesive or the like properties resulting from absorption of a small amount of water or moisture. These water-absorbent amino acids are proline, lysine, histidine, cysteine, ornithine, glutamic acid, and arginine.

These amino acids can be obtained by fermentation method, synthetic method, and plant extraction method, etc., and may be used solely or in combination of two or more kinds of such amino acids.

In addition, the water-absorbent amino acids used in the present invention may be a salt, and examples of such salts include salts with an organic or inorganic acid such as hydrochloride, sulfate, and acetate, and salts with a basic salt such as sodium salt, potassium salt, and the like. The water-absorbent amino acids preferably constitute amino acid granules which constitute a tablet, wherein the water-absorbent amino acid(s) is/are contained in preferably about 30 to 98.5 % by mass, more preferably about 35 to 90 % by mass, most preferably about 35 to 80 % by mass to the total mass of the above-mentioned amino acid granules. In addition, the water-absorbent amino acids are contained in preferably about 10 to 70 % by mass, more preferably 20 to 70 % by mass, most preferably 20 to 60 % by mass to the total mass of a tablet.

A coating agent used in the present invention is soluble in ethanol as well as hardly soluble in water. In this case, "hardly soluble" means "very slightly soluble" or "practically insoluble or insoluble" as described in the terms of solubility in The Japanese Pharmacopoeia Fourteenth Edition. On the other hand, "soluble in ethanol" includes the case of not only "clearly dissolved in ethanol" but also "emulsifiable in ethanol". The coating agents are shellac and zein.

In the present invention, a coating agent which is soluble in ethanol and hardly soluble in water may be blended with the water-absorbent amino acid in any form. However, the coating agent which is soluble in ethanol and hardly soluble in water is preferably blended in such a way that the coating agent coats the water-absorbent amino acids. In the case where the water-absorbent amino acids constitute amino acid granules which constitute the tablet, the coating agent which is soluble in ethanol and hardly soluble in water is blended in such a manner that the coating agent coats the above-mentioned amino acid granules. The tablet is constituted by amino acid granules coated with a coating agent which is soluble in ethanol and hardly soluble in water.

With respect to the above-mentioned amino acid granules, the coating agent is preferably about 1 to 6 % by mass, more preferably 2 to 5 % by mass, to the total mass of the granules. With respect to the tablet mass of the present invention, the coating agent is preferably about 0.1 to 4.2 % by mass, more preferably about 0.2 to 4.2 % by mass, most preferably about 0.5 to 4.2 % by mass, relative to the total tablet mass.

As for the tablet in the present invention or the above-mentioned amino acid granules coated by a coating agent which is soluble in ethanol and hardly soluble in water, there can be employed conventional excipients used in conventional nutritional supplements or pharmaceuticals, such as saccharides and sugar alcohols besides the water-absorbent amino acids which are main active ingredient.

Examples of the saccharides include monosaccharides and disaccharides, and more specific examples include lactose, maltose, trehalose and the like. Examples of the sugar alcohols include mannitol, hydrogenated maltose starch syrup, maltitol, maltol, lactitol, xylitol, sorbitol, erythritol and the like. Such saccharides or sugar alcohols can be optionally selected from one or, two or more kinds of combinations thereof, depending on the kind, the blended ratio, and the content of water absorbent amino acid, and the like. In addition, saccharides or sugar alcohols can be contained solely or in combination thereof in an amount of about 15 to 85 % by mass, more preferably about 15 to 60 % by mass, most preferably about 20 to 40 % by mass to the total mass content of the tablet of the present invention. Moreover, such saccharides or sugar alcohols can be contained alone or in combination thereof in an amount of 1 to 65 % by mass, more preferably about 1 to 40 % by mass, most preferably about 3 to 25 % by mass, to the total mass content of the above-mentioned water-absorbent amino acid granules coated by a coating agent which is soluble in ethanol and hardly soluble in water.

In order to give disintegrability in the oral cavity to the tablets of the present invention, such tablets or water-absorbent amino acid granules coated by a coating agent which is soluble in ethanol and hardly soluble in water according to the present invention may contain an intraoral disintegration-promoting ingredient. Examples of the above-mentioned intraoral disintegration-promoting ingredient include carboxymethylcellulose, calcium carboxymethylcellulose, sodium carboxymethylcellulose, crospovidone, croscarmellose sodium, sodium starch glycolate, and the like, more preferably carboxymethylcellulose or sodium starch glycolate. These oral disintegration promoting agents can be used alone or in combination thereof in an amount of about 0.5 to 20 % by mass, more preferably about 0.5 to 5 % by mass, most preferably about 0.5 to 2 % by mass, relative to the total mass content of the tablet of the present invention or the above-mentioned amino acid granules coated by a coating agent which is soluble in ethanol and hardly soluble in water.

Moreover, the intraoral disintegrability of the tablet of the present invention may be improved in combination of the tablet of the present invention or the above-mentioned amino acid granules coated by a coating agent which is soluble in ethanol and hardly soluble in water, and a freely water soluble sugar alcohol such as mannitol, sorbitol, erythritol, xylitol, lactitol. In such case, the above-mentioned intraoral disintegration-promoting ingredient may not necessarily be added thereto.

Furthermore, tablets of the present invention or the above-mentioned amino acid granules coated by a coating agent which is soluble in ethanol and hardly soluble in water, can optionally contain binders, lubricants, other additives, and the like.

Examples of the binders include methylcellulose, ethylcellulose, polyvinylpyrrolidone, pullulan, acrylate-type polylmer, polyvinyl alcohol, gelatin, agar, acacia, powdered acacia, partly pregelatinized starch, macrogol, and the like, and one or, two or more kinds of these binders can be optionally used. The binders can contain preferably about 0.1 to 5 % by mass, more preferably about 0.3 to 3 % by mass, most preferably about 0.5 to 2 % by mass, to the total content of the tablet of the present invention or the above-mentioned amino acid granules coated by a coating agent which is soluble in ethanol and hardly soluble in water.

Examples of the lubricants include, sucrose esters of fatty acid, magnesium stearate, calcium stearate, sodium stearyl fumarate, talc, sodium lauryl sulfate, light anhydrous silicic acid, hydrous silicon dioxide, sucrose esters of fatty acid and the like, and among these, one or, two or more kinds of these lubricants may be optionally used. The lubricants can be contained in an amount of preferably about 0.05 to 10 % by mass, more preferably about 0.1 to 5 % by mass, most preferably 0.1 to 3 % by mass, to the total content of the tablet of the present invention or the above-mentioned amino acid granules coated by a coating agent which is soluble in ethanol and hardly soluble in water.

In addition, amino acids other than the water-absorbent amino acids may be contained in the tablet of the present invention or the above-mentioned amino acid granules coated by a coating agent which is soluble in ethanol and hardly soluble in water.

Examples of the amino acids other than the water-absorbent amino acids include branched chain amino acids (e.g. valine, leucine, isoleucine, etc.), glutamine, tyrosine, tryptophan, alanine, methionine, cysteine, phenylalanine, glycine, aspartic acid, serine, and the like. The amino acids other than the water-absorbent amino acids can be optionally selected from one or, two or more kinds of combinations thereof, depending on the kind, the blended ratio, and the content of water-absorbent amino acids. The amino acids other than the water-absorbent amino acids can be contained alone or in combination thereof, in an amount of preferably about 5 to 60 % by mass, more preferably about 10 to 50 % by mass, most preferably about 15 to 40 % by mass, to the total content of the tablet of the present invention. Moreover, the amino acids other than the water-absorbent amino acids can be contained alone or in combination thereof, in an amount of preferably about 1 to 65 % by mass, more preferably about 15 to 65 % by mass, most preferably about 30 to 60 % by mass, to the total content of the above-mentioned water-absorbent amino acid granules coated by a coating agent which is soluble in ethanol and hardly soluble in water.

Examples of other additives include a carbohydrate such as dextrin and starch; corrigents for bitter taste such as cyclodextrin; colorants such as β-carotene, food dye (e.g. Food Yellow No.5, Food Red No. 2 , Food Blue No.2, etc.), food lake dye, red ferric oxide, and niacin; a vitamin or derivatives thereof such as vitamin E, ascorbic acid, vitamins B, vitamin A, and vitamin D; minerals such as sodium; sweeteners such as aspartame, glucose, fructose, sucralose, stevia, saccharose, saccharin sodium, thaumatin, and acesulfame potassium; a glidants such as fine silicic dioxide, calcium silicate, synthetic aluminum silicate, and talc; foaming agents such as sodium bicarbonate; acid such as citric acid, malic acid, and tartaric acid; a flavor such as lemon, lemon lime, orange, and menthol; cellulose or its derivative; a crystalline cellulose; and a microcrystalline cellulose: and the like, and among these, one or, two or more kinds of these substances may be optionally used. Other additives may be contained in an amount of preferably about 0.01 to 5 % by mass, more preferably about 0.1 to 3 % by mass, most preferably about 0.1 to 1 % by mass, relative to the total content of the tablet of the present invention or the above-mentioned amino acid granules coated by a coating agent which is soluble in ethanol and hardly soluble in water.

In the present invention, the tablet may be any one provided that they are used as nutritious food and pharmaceuticals, and examples of such tablets include a conventional tablet which is dissolved in the stomach, an enteric tablet, a chewable preparation, a troche, and an intraorally rapidly disintegrable tablet and the like. The chewable preparation or an intraorally rapidly disintegrable tablet is preferable, because water-absorbent amino acids are capable of being supplemented by simple administration method. In addition, in order to improve moisture proof property and storage stability, the tablet may be a sugar coated tablet or a coated tablet by saccharide, hydrogenated maltose starch syrup, and sugar alcohol, etc.

It is preferable that the tablet of the present invention contains preferably about 10 to 80 % by mass of the above-mentioned amino acid granules coated with a coating agent which is soluble in ethanol and hardly soluble in water, relative to the total mass of the tablet. However, in order to achieve a high function for the use of an amino acid as nutrition, the amino acid granules may be contained in an amount of preferably about 30 to 85 % by mass, more preferably about 35 to 70 % by mass, most preferably about 40 to 65 % by mass. Since the tablets of the present invention are comprised of the amino acid granules, the above-mentioned excipients, the oral disintegration promoting agents, the binders, the lubricants, the amino acids other than the water-absorbent amino acids, and other additives, etc. which are contained in the tablet of the present invention or the above-mentioned amino acid granules coated with a coating agent which is soluble in ethanol and hardly soluble in water, may be preferably contained inside and/or outside the said amino acid granules, and the amino acids other than the water-absorbent amino acids may be contained inside the amino acid granules. In addition, the said excipients may be preferably contained outside the said amino acid granules.

In the case where the above-mentioned additives, etc. are previously contained in the above-mentioned amino acid granules, it is preferable that the sum of each additive content contained in the amino acid granules previously contained and the content of the additive which is further added outside the amino acid granules, are within the above-mentioned preferable range of % by mass to the total tablet mass.

In the case where the tablet of the present invention is used as a chewable preparation, the tablet diameter is preferably about 7 to 20 mm, more preferably about 9 to 17 mm, further more preferably about 10 to 16 mm so as to be easily chewed. The tablet mass is preferably about 300 mg to 1.5 g, more preferably about 400 mg to 1 g, further more preferably about 500 to 900 mg. With respect to the above-mentioned chewable preparation, the content of water-absorbent amino acids can be increased and, in this case, tablet mass content can be decreased to that of the water absorbent amino acids. As a result, the thickness of the tablet relative to the diameter of the tablet can be reduced. The chewable preparation thickness of the present invention is preferably about 2 to 8 mm, more preferably about 3 to 6 mm, most preferably about 3.5 to 5 mm.

In addition, in the case where the tablet of the present invention is used as chewable preparation, such tablet is preferably softer than common tablets, and its hardness is preferably about 50 to 120 N, more preferably about 60 to 110 N, still more preferably about 65 to 100 N, and most preferably about 70 to 90 N.

Moreover, in the case where the tablet hardness is not higher than 50 N, it can be used as an intraorally rapidly disintegrable tablet.

The upper limit of the tablet hardness is usually about 120 N, wherein the hardness is determined in the diameter direction of the tablet using a hardness tester (manufactured by Japan Machinery Co., Type PTB-301).

Hereinafter, preferable preparation method of the tablet of the present invention is described.
(1) Manufacturing of amino acid granules coated with a coating agent which is soluble in ethanol and hardly soluble in water.
   A powder containing one or more members of proline, lysine, histidine, cysteine, ornithine, glutamic acid and arginine, and a salt thereof, and containing a water-absorbent amino acid(s) in an amount of preferably not less than about 10 % by mass, more preferably not less than about 20 % by mass, most preferably not less than about 30 % by mass, to the whole is used. The powder which has an average particle diameter of preferably not more than about 300 µm, more preferably not more than about 200 µm is used as an ingredient. This ingredient is supplied into a granulator such as an agitation granulator, a high-speed agitation granulator, a fluidized bed granulator dryer, an extrusion granulator, or a tumbling fluidized bed granulator, preferably a fluidized bed granulator dryer. If necessary, water (binding water) containing one or more substances selected from methylcellulose, mannitol, xylitol, erythritol, ethylcellulose, and carboxymethylcellulose, and the like in an amount of about 0.5 to 5 % by mass, more preferably about 1 to 4 % by mass, and most preferably about 1.5 to 3.5 % by mass of water (binding water) may be sprayed, and then granulation is performed in the usual manner. It is preferable that the water used herein is water such as purified water provided that it is acceptable in view of the Food Sanitation Law, or Pharmaceutical Affairs Law of Japan.
   In addition, in the case where the above-mentioned excipients such as saccharides or sugar alcohols, intraorally disintegration promoting ingredients, binders, lubricants, other additives or the like is previously combined, it is preferable to granulate using a ingredient obtained by combining and admixing the desired content of the above-mentioned additives, etc. with the above pulverized water-absorbent amino acid powder.
   Then, the coating of the resulting granules (the average particle diameter is preferably about 75 to 850 µm), is performed by spraying an ethanol solution in which a coating agent which is soluble in ethanol and hardly soluble in water ( shellac or zein ) is dissolved in an amount of preferably about 5 to 20 % by mass, more preferably about 7 to 15 % by mass of the resulting granules (the average particle diameter is preferably about 75 to 850 µm), with the use of a fluidized bed granulator equipped with a spray apparatus, a coating machine equipped with a spray apparatus, etc. On this occasion, although nozzle diameter and spray rate may depend on such equipment and spray quantity, it is preferable to use a spray apparatus with a nozzle bore size of about 0.8 to 2.2 mmφ and to perform the spraying at the rate of about 20 to 300 mL/minute. The above coating procedure gives amino acid granules which are preferably coated with about 1 to 6 % by mass, more preferably 2 to 5 % by mass, of a coating agent which is soluble in ethanol and hardly soluble in water, relative to the total amount of the granules.
(2) Method for manufacturing tablets which contain a water-absorbent amino acid and a coating agent which is soluble in ethanol and hardly soluble in water.

As a method for manufacturing tablets of the present invention, such method includes a compression molding method for the amino acid granules which are coated with a coating agent which is soluble in ethanol and hardly soluble in water.

There is no particular limitation for the compression molding method, and conventionally known methods may be used. Examples of such method include a common method in which various additives as mentioned above are, if required, added to amino acid granules coated with a coating agent which is soluble in ethanol and hardly soluble in water manufactured by the above (1), followed by mixing and compression molding, and a method in which a lubricant is previously applied on the surface of punch and on the die wall, and then amino acid granules coated with a coating agent which is soluble in ethanol and hardly soluble in water and various additives other than the lubricant are mixed, and compression molded.

Here, excipients, oral disintegration promoting agents, binders, lubricants, other additives, etc. may be independently in the form of powder or liquid, or they may be in the form of granules which are previously granulated in combination of such additives by the known method such as wet granulation and dry granulation.

Examples of the method for granulating the above-mentioned various additives include a method in which acceptable water in view of the Food Sanitation Law or the Pharmaceutical Affairs Law of Japan, such as purified water, is added to the above-mentioned various additives, followed by kneading and granulating, and a method in which water is sprayed to the above-mentioned various additives for the granulation. With respect to such kneading and granulating method, in the case where the additive, etc. contain a liquid such as sugar alcohol, all or part of the above-mentioned water may be optionally replaced by a sugar alcohol. In the granulation by spraying, such granulation may be carried out according to, for example, a granulation method used in the manufacture of amino acid granules coated with a coating agent which is soluble in ethanol and hardly soluble in water, as described in the above (1), e.g. a granulation method with a drying fluidized bed granulator.

It is preferable that the resulting tablets are further dried. Drying may be performed by any method which is commonly used as a means for manufacturing preparations, such as vacuum drying, freeze drying, and natural drying.

In addition, besides the method for manufacturing tablets containing water-absorbent amino acid and a coating agent which is soluble in ethanol and hardly soluble in water as described in the above (2), for example, tablets of the present invention can also be manufactured by a process comprising adding a saccharide or a sugar alcohol to amino acid granules coated with a coating agent which is soluble in ethanol and hardly soluble in water, applying a strong pressure to the resulting mixture as it is in a dry state, into a massive product, crushing the massive product into powdery granules and compression molding the granules (additives may be added in this procedure).

There is no limitation on the apparatus used for tableting, and an apparatus which is commonly used for molding or granulation of tablets may be used. For example, a rotary tableting machine, a single-punch tableting machine, etc. may be used. The tableting pressure when tableted is preferably about 1 to 100 kN/cm², more preferably about 5 to 50 kN/cm², and most preferably about 10 to 40 kN/cm².

Besides the objective effects of muscular-fatigue reduction, fatty acid burn, dieting, etc., the tablets of the present invention can be used as nutritional supplements or pharmaceuticals and the like in order to supply well-balanced nutrition effective to human health maintenance and activate human tissues physiologically, etc. Moreover, subjects to which the tablet is administered is mainly mammals such as human beings, and administration method is a comparatively easy method, for example, preferably oral administration. Furthermore, although the dosage of the tablet of the present invention depends upon administration purpose, and sex, body weight and age of the subjects to be administered such as human beings and the like, is not necessarily appropriate to suggest, the dosage is usually about 0.1 to 20 g/day, preferably about 0.2 to 20 g/day, in particular preferably about 0.2 to 10 g/day.

In the present invention, the tablet may comprise the water-absorbent amino acid and a coating agent which is soluble in ethanol and hardly soluble in water acceptable under the Food Sanitation Law or the Pharmaceutical Affairs Law of Japan as its main constituent component, and methyl cellulose, mannitol, etc., which are used in the granulation of water-absorbent amino acids, are included therein and additives such as excipients or lubricants which are optionally included as for other components. These additives are preferably acceptable under the Food Sanitation Law or the Pharmaceutical Affairs Law of Japan, too.

The following Examples are merely preferred embodiments of the present invention.

### Examples

### Example 1

With the use of a fluidized bed granulator for coating (Flow Coater (model type: FL-LABO) manufactured by Freund Corporation), 350g of an amino acid mix powder (manufactured by Kyowa Hakko Kogyo Co., Ltd.) containing 3.74 % by mass of glutamic acid, 16.5 % by mass of proline, 11.4 % by mass of glycine, 12.53 % by mass of lysine hydrochloride, and 4.87 % by mass of arginine was granulated by spraying 100 mL of an aqueous solution containing 2.8 g of methyl cellulose (type:SM-100, manufactured by Shin-Etsu Chemical Co., Ltd.) in 200 mL of water, thereby to prepare granules having an average diameter of 250 to 300 µm. Subsequently, in the same apparatus, an ethanol solution which contains 10.5 g of shellac in 150 mL of ethanol was all sprayed on the resulting granules to obtain Granule 1 which was coated by 3 % by mass of shellac.

The above process was repeated several times, and to the resulting Granule 1 (6000 g) were added 3200 g of erythritol, 200 g of calcium carboxymethylcellulose, 500 g of sucrose fatty acid ester, and 100 g of orange flavor, followed by mixing.

After that, the above mixture was subjected to compression molding with tableting pressure of 20 kN using a rotary tableting machine (trade name: type AP-15; manufactured by Hata Iron Works Co., Ltd.) equipped with a plane punch of 13 mm diameter to prepare chewable tablets (hereinafter, referred to as tablet 1), each weighing 600 mg (containing 360 mg of amino acids; amino acid content: about 60% by weight).

### Example 2

With the use of a fluidized bed granulator for coating (Flow Coater (model type: FLO-5) manufactured by Freund Corporation), 3000 g of an amino acid mix powder (manufactured by Kyowa Hakko Kogyo Co., Ltd.) containing 3.74 % by mass of glutamic acid, 16.5 % by mass of proline, 11.4 % by mass of glycine, 12.53 % by mass of lysine hydrochloride, and 4.87 % by mass of arginine was granulated by spraying 1.3 L of an aqueous solution which contains 21 g of methylcellulose (Type: SM-100, Shin-Etsu Chemical Co., Ltd.) in 1.5 L of water, thereby to prepare granules having an average diameter of 250 to 300 µm. Subsequently, in the same apparatus, an ethanol solution containing 90 g of shellac in 1.3 L of ethanol was all sprayed on the resulting granules to obtain Granule 2 which was coated by 3 % by mass of shellac.

The above process was repeated several times, and chewable tablets (hereinafter referred to as Tablet 2) were prepared similarly to Working Example 1, using the resulting Granules 2 (6000g).

### Evaluation test of tablets

The physical properties (hardness and intraoral disintegration time) of the obtained Tablets 1 and 2 were evaluated. As a result, both tablets showed tablet hardness of about 70 N and intraoral disintegration time of about 90 seconds, and such tablets have an excellent mouthfeel as a chewable preparation. In addition, tableting problems such as capping, sticking and like in the tableting process was not observed, and continuous tableting for 4 hours was able to be performed.

### Comparative Example

An amino acid mix powder (manufactured by Kyowa Hakko Kogyo Co., Ltd.) containing 3.74 % by mass of glutamic acid, 16.5 % by mass of proline, 11.4 % by mass of glycine, 12.53 % by mass of lysine hydrochloride, and 4.87 % by mass of arginine was tried to be tableted without coating with a coating agent which is soluble in ethanol and hardly soluble in water, but even test tableting was impossible.

The tablets of the present invention are useful as amino acid preparations containing water-absorbent amino acids for sports, dieting, and pharmaceuticals.

## Claims

1. A compression-molded tablet comprising water-absorbent amino acid granules coated with shellac or zein as a coating agent, wherein the water-absorbent amino acid is at least one amino acid seceded from proline, lysine, histidine, cysteine, ornithine, glutamic acid and arginine.

2. The tablet according to claim 1, wherein the tablet comprises the water-absorbent amino acid in an amount of 10 to 70 % by mass of the total tablet mass.

3. The tablet according to claim 1 or 2, wherein the tablet comprises the coating agent in an amount of 1 to 6 % by mass of the total mass of the granules.

4. The tablet according to any one of claims 1 to 3, wherein the tablet is a chewable preparation.

5. A method for manufacturing a tablet containing a water-absorbent amino acid, which comprises a step of granulating a powder containing a water-absorbent amino acid, wherein the water-absorbent amino acid is at least one amino acid selected from proline, lysine, histidine, cysteine, ornithine, glutamic acid and arginine, coating the resulting granules by spraying an ethanol solution containing 5 to 20 by mass of shellac or zein as a coating agent, and compression molding the resulting coated granules.

6. The method according to claim 5, wherein the water-absorbent amino acid is used in an amount of 10 to 70 % by mass of the total tablet mass.

7. The method according to claim 5 or 6, wherein the coating agent is used in an amount of 1 to 6 % by mass of the total mass of the granules.

8. The tablet according to any of claims 1 to 4 for reducing muscular-fatigue.

9. The tablet according to any of claims 1 to 4 for burning fatty acid.

10. The tablet according to any of claims 1 to 4 for dieting.

## Patentansprüche

1. Formgepresste Tablette, umfassend wasserabsorbierende Aminosäuregranulatkörner, die mit Schellack oder Zein als ein Überzugsmittel überzogen sind, wobei die wasserabsorbierende Aminosäure mindestens eine Aminosäure, ausgewählt aus Prolin, Lysin, Histidin, Cystein, Omithin, Glutaminsäure und Arginin, ist.

2. Tablette nach Anspruch 1, wobei die Tablette die wasserabsorbierende Aminosäure in einer Menge von 10 bis 70 Gew.-% des Gesamtgewichts der Tablette umfasst.

3. Tablette nach Anspruch 1 oder 2, wobei die Tablette das Überzugsmittel in einer Menge von 1 bis 6 Gew.-% des Gesamtgewichts der Granulatkörner umfasst.

4. Tablette nach einem der Ansprüche 1 bis 3, wobei die Tablette eine kaubare Zubereitung ist.

5. Verfahren zur Herstellung einer Tablette, die eine wasserabsorbierende Aminosäure enthält, umfassend einen Schritt des Granulierens eines Pulvers, welches eine wasserabsorbierende Aminosäure enthält, wobei die wasserabsorbierende Aminosäure mindestens eine Aminosäure, ausgewählt aus Prolin, Lysin, Histidin, Cystein, Ornithin, Glutaminsäure und Arginin, ist, des Überziehens der resultierenden Granulatkörner durch Sprühen einer Ethanollösung, die 5 bis 20 Gew.-% Schellack oder Zein als ein Überzugsmittel enthält, und des Formpressens der resultierenden überzogenen Granulatkörner.

6. Verfahren nach Anspruch 5, wobei die wasserabsorbierende Aminosäure in einer Menge von 10 bis 70 Gew.-% des Gesamtgewichts der Tablette verwendet wird.

7. Verfahren nach Anspruch 5 oder 6, wobei das Überzugsmittel in einer Menge von 1 bis 6 Gew.-% des Gesamtgewichts der Granulatkörner verwendet wird.

8. Tablette nach einem der Ansprüche 1 bis 4 zur Reduktion von Muskelermüdung.

9. Tablette nach einem der Ansprüche 1 bis 4 zur Verbrennung von Fettsäuren.

10. Tablette nach einem der Ansprüche 1 bis 4 zur Diät.

## Revendications

1. Comprimé moulé par compression, comprenant des granulés d'acide aminé hydro-absorbant, enrobés avec un agent d'enrobage qui est du shellac ou de la zéine, dans lequel ledit acide aminé hydro-absorbant est au moins un acide aminé choisi parmi les proline, lysine, histidine, cystéine, ornithine, acide glutamique et arginine.

2. Comprimé conforme à la revendication 1, ledit comprimé comprenant l'acide aminé hydro-absorbant en une quantité représentant 10 à 70 % de la masse totale du comprimé.

3. Comprimé conforme à la revendication 1 ou 2, ledit comprimé comprenant l'agent d'enrobage en une quantité représentant 1 à 6 % de la masse totale des granulés.

4. Comprimé conforme à l'une des revendications 1 à 3, ledit comprimé étant une préparation à mâcher.

5. Procédé de fabrication d'un comprimé contenant un acide aminé hydro-absorbant, ledit procédé comprenant les étapes de granulation d'une poudre contenant un acide aminé hydro-absorbant, ledit acide aminé hydro-absorbant étant au moins un acide aminé choisi parmi les proline, lysine, histidine, cystéine, ornithine, acide glutamique et arginine, d'enrobage des granulés résultants par pulvérisation d'une solution contenant, dans de l'éthanol, 5 à 20 % en masse d'un agent d'enrobage qui est du shellac ou de la zéine, et de moulage par compression des granulés enrobés ainsi obtenus.

6. Procédé conforme à la revendication 5, dans lequel on emploie l'acide aminé hydro-absorbant en une quantité représentant 10 à 70 % de la masse totale du comprimé.

7. Procédé conforme à la revendication 5 ou 6, dans lequel on emploie l'agent d'enrobage en une quantité représentant 1 à 6 % de la masse totale des granulés.

8. Comprimé conforme à l'une des revendications 1 à 4, conçu pour réduire la fatigue musculaire.

9. Comprimé conforme à l'une des revendications 1 à 4, conçu pour faire brûler les acides gras.

10. Comprimé conforme à l'une des revendications 1 à 4, conçu en vue d'un régime.
